# EUROPEAN PATENT SPECIFICATION

(11) **EP 0 733 034 B1**
(45) Date of publication and mention of the grant of the patent: **02.09.1998**
(21) Application number: 95902857.2
(22) Date of filing: 06.12.1994
(51) Int. Cl.: C07C 67/38, C07C 69/54

(54) **PROCESS FOR THE CARBONYLATION OF AN ACETYLENICALLY UNSATURATED COMPOUND**
VERFAHREN ZUM CARBONYLIEREN EINER ACETYLENISCH UNGESÄTTIGTEN VERBINDUNG
PROCEDE DE CARBONYLATION D'UN COMPOSE ACETYLENIQUEMENT INSATURE

(30) Priority: 10.12.1993 GB 9325377
(43) Date of publication of application: 25.09.1996
(73) Proprietor: IMPERIAL CHEMICAL INDUSTRIES PLC, London SW1P 3JF (GB)
(72) Inventor: TOOZE, Robert Paul, Stockton-on-Tees, Cleveland TS20 1EA (GB)
(74) Representative: Nevard, Edward John
(86) International application number: GB9402667
(87) International publication number: WO9515938

(56) References cited:
- EP-A- 0 055 875
- EP-A- 0 186 228
- EP-A- 0 495 547

## Description

The invention relates to a process for the carbonylation of an acetylenically unsaturated compound with carbon monoxide in the presence of water, an alcohol and/or carboxylic acid/

It is known that such a compound may be carbonylated with carbon monoxide in the presence of water, an alcohol and/or carboxylic acid to yield carboxylic acids, esters or anhydrides respectively.

European Patent EP 0194707 B discloses the hydrocarboxylation of an acetylenically unsaturated compound with carbon monoxide and a carboxylic acid in the liquid phase. The reaction is shown to proceed at a temperature of 115°C in the presence of a catalyst system prepared from a palladium (II) compound and a triorganic phosphine, wherein the ratio of the number of moles of triorganic phosphine to gram atom of palladium (II) is greater than 15. The most effective of those triorganic phosphines described, in terms of reaction rate, is triphenyl phosphine.

Published European Patent Application EP 0271144 A discloses the carbonylation of an acetylenically unsaturated compound with carbon monoxide in the presence of a hydroxyl-containing compound, e.g. water, an alcohol, a phenol and/or carboxylic acid, in the liquid phase. The reaction is shown to proceed at a temperature from 40 to 90°C in the presence of a catalyst system prepared from a palladium (II) compound and a triorganic phosphine compound wherein at least one of the organic groups is a heterocyclic nitrogen containing group, e.g. pyridyl. One of the most effective of the phosphines described is diphenyl-2-pyridylphosphine. A comparison of the results disclosed in EP 0194707 B with those in EP 0271144 A show that the triorganic phosphine compounds which contain a heterocyclic nitrogen containing group are ten or more times reactive as the previous triorganic compounds, even at lower temperatures.

A suitable feedstock which contains an acetylenically unsaturated compound may be obtained from the cracking of oil fractions. Such a feedstock frequently contains an allenically unsaturated compound in addition to the acetylenically unsaturated compound. It is known, however, that an allenically unsaturated compound can have a detrimental effect on the catalyst system as described in EP 0271144 A. Consequently, the use of such a catalyst system requires the elimination of any allenically unsaturated compound from the feedstock prior to carbonylation thereof. The elimination of the allenically unsaturated compounds requires the use of additional feedstock preparation stages, e.g. isomerization and separation, which add to the overall expense and complexity of the conventional carbonylation process. It is therefore desirable to dispense with the additional feedstock preparation stages by, for example, the use of an alternative more tolerant catalyst system. However, the other known catalyst systems based on triphenyl phosphine show such low activity at temperatures below their respective decomposition points under hydrocarbonylation conditions and particularly below 100°C that they cannot be viewed as practical substitutes for the catalyst systems based on pyridyl phosphine.

It is therefore an object of the present invention to produce a catalyst system which may be of practical use in the carbonylation of an acetylenically unsaturated compound at relatively low temperatures and which catalyst system is at least tolerant to the presence of an allenically unsaturated compound.

Accordingly, the present invention provides a process capable of the low temperature carbonylation of an acetylenically unsaturated compound with carbon monoxide in the presence of an allenically unsaturated compound and a catalytic system which is capable of catalysing the carbonylation of the acetylenically unsaturated compound and which is at least tolerant of the allenically unsaturated compound, the catalytic system being formed from
(a)a palladium compound;
(b)a protonic acid;
(c)an organic phosphine of the form (I) 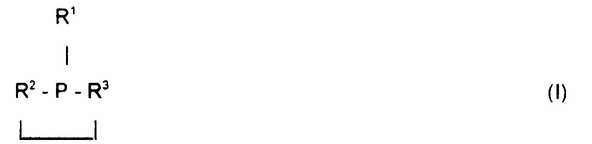
wherein
R¹ is an optionally substituted aryl group;
R² and R³ are joined to form an optionally substituted cyclic moiety comprising at least one optionally substituted alkylene group.

The mechanism through which the catalytic system tolerates the presence of the allenically unsaturated compound and enhances the rate of carbonylation of the acetylenically unsaturated compound is not fully understood. However, it is believed that the cyclic moiety may act to provide a source of electrons and/or to regulate the access of unsaturated compounds to the catalyst. It is therefore preferred that the cyclic moiety is a relatively rigid structure which contains from 4 to 15, particularly from 6 to 13, and especially from 8 to 11, optionally substituted alkylene groups. The phosphorus atom and the cyclic moiety join to form a mono or polycyclic structure. Thus, the organic phosphine is one in which the phosphorus atom forms a link within at least one cyclic group. A particularly preferred organic phosphine is one in which the phosphorus atom, R² and R³ form a polycyclic group in which the phosphorus atom is a link on a bridge shared by at least two cyclic groups. Particularly preferred polycyclic groups include 9-phosphabicyclo[4.2.1]-nonane and 9-phosphabicyclo[3,3,1]-nonane. Especially preferred is the [4.2,1] isomer.

The group R¹ is an optionally substituted aryl group. Preferred groups include anthryl, naphthyl and phenyl all of which may be optionally substituted. Particularly preferred is optionally substituted phenyl and especially preferred is phenyl.

The palladium compound and protonic acid may be any used in EP 0194707 B and EP 0271144 A. Suitable combinations employ palladium acetate with a protonic acid such as phenylphosphonic acid or methanesulphonic acid.

The quantity of palladium compound used can be varied within a wide range. Typically a molar ratio from 10⁻⁶:1 to 1:1, preferably from 10⁻⁵:1 to 1:1 and particularly from 10⁻⁵:1 to 10⁻²:1 of palladium to unsaturated compound may be used.

The quantity of organic phosphine to palladium compound can also vary within a wide range. Typically a ratio from 200:1 to 1:1 of organic phosphine to palladium compound may be used.

The quantity of protonic acid used is not critical and can be varied within a wide range. Typically a ratio from 200:1 to 0.1 of protonic acid to organic phosphine may be used.

Suitable acetylenically unsaturated compounds which may be used in the present invention include one or more unsaturated alkyne having from 2 to 20 carbon atoms, for example ethyne, propyne, 1-butyne, 2-butyne, 1-pentyne, 1-hexyne, 1-heptyne, 1-octyne, 2-octyne, 4-octyne, 5-methyl-3-heptyne, 4-propyl-2-pentyne, 1-nonyne, benzylethyne and cyclohexylethyne. The acetylenically unsaturated compound may also be substituted by one or more groups or atoms for example halo, cyano, ester alkoxy, aryl and hydroxy groups.

Suitable allenically unsaturated compounds which may be used in the present invention include one or more alkadiene having from 3 to 20 carbon atoms, for example propadiene (allene), 1, 2-butadiene, 1, 2-pentadiene, 3, 4-octadiene and 3-methyl-1, 2-butadiene. The allenically unsaturated compound may also be substituted by one or more groups or atoms, such substituents include halo, cyano, ester, alkoxy, aryl and hydroxy groups.

The catalyst systems used in the present invention may not only tolerate the presence of allenically unsaturated compounds, but adventitiously may also carbonylate such compounds. Thus, when R¹ is phenyl and R² and R³ represent the preferred polycyclic groups then such a catalyst system shows a propensity for carbonylating allene to methyl methacrylate under the same conditions used for the carbonylation of, for example, propyne

The carbonylation process of the present invention may use any suitable source of hydroxy groups, for example water, an alcohol or carboxylic acid, or mixtures thereof. Suitably the hydroxy groups are provided by an alcohol, e.g. methanol.

The carbonylation process of the present invention may also be conducted in the presence of a suitable solvent. Such solvents include anisole and diphenyl ether. Other solvents include those disclosed in the prior art acknowledged supra.

The present catalyst system may be used at elevated temperatures, for example from 115°C to 150°C. However, the present catalyst system adventitiuosly allows the use of relatively low temperatures as compared to those conventionally employed when triphenylphosphine is used as the organic phosphine compound. Typically, therefore, the temperature at which the carbonylation is performed is less than 115°C, preferably less than 100°C, for example from 20 to 90°C, and is preferably from 40 and 90°C and especially from 50 to 90°C, for example from 50 to 70°C.

The pressure under which the carbonylation is performed may be selected by the skilled person in order to suit the particular catalyst system used. Typically, a total pressure from 5 to 100 bar, and in particular from 30 to 80 bar may be used.

The present invention is illustrated by reference to the following examples.

### EXAMPLE 1

A 1 litre capacity Hastalloy C autoclave was evacuated and then charged with a catalyst solution comprising palladium acetate (0.8 mmol), phosphine ligand (9-phenyl 9-phosphabicyclo nonane (PPBN) (40 mmol), phenylphosphonic acid (40 mmol), methanol (40 cm³) and anisole (160 cm³).

The PPBN used was a mixture of the [3,3,1] and [4,2,1] isomers which were present in the approximate molar ratio of 1:4.

Methyl acetylene (30 g) was then introduced and the autoclave pressurised to 60 bar with carbon monoxide and heated to 60°C.

After set times samples were removed from the autoclave and analysed by gas chromatography for methyl methacrylate.

### EXAMPLE 2 - COMPARATIVE

Example 1 was repeated except that triphenyl phosphine was used as the phosphine ligand instead of PPBN. The results of Examples 1 and 2 are shown in Table 1 as the percentage (w/v) of methyl methacrylate detected in the withdrawn samples.

**TABLE 1**

| LIGAND | REACTION TIME (HOURS) | |
|---|---|---|
| | 2 | 24 |
| PPBN | 0.5 | 1.6 |
| Triphenyl phosphine | 0.0 | 0.4 |

To further compare the effect of the different ligands the relative rates at which the feedstock was consumed were calculated in terms of moles of feed consumed per mole of palladium per hour (Relative Rate). The Relative Rates obtained from Examples 1 and 2 are shown in Table 1a below.

**TABLE 1a**

| LIGAND | REACTION TIME (HOURS) | |
|---|---|---|
| | 2 | 24 |
| PPBN | 8.5 | 7.3 |
| Triphenyl phosphine | 0.0 | 0.5 |

It is thus evident that the conventional phosphine ligand is much less active than PPBN under low temperature conditions.

### EXAMPLE 3

Examples 1 and 2 were repeated except that in respect of the PPBN, two experiments were conducted using different amounts of palladium acetate, phenylphosphonic acid and PPBN. The amounts of these components were as follows (i) 0.1 g palladium acetate in association with 3.6 g of phenylphosphonic acid and 5.0 g of PPBN and (ii) 0.05 g palladium acetate in association with 1.84 g of phenylphosphonic acid and 2.6 g of PPBN. In both experiments the results were approximately the same.

In terms of the Relative Rates the results are as shown in Table 2.

**TABLE 2**

| LIGAND | REACTION TIME (HOURS) | |
|---|---|---|
| | 2 | 24 |
| PPBN | 6.3 | 5.3 |
| Triphenyl phosphine | 0.0 | 0.5 |

### EXAMPLE 4

Example 1 was repeated except that the propyne was replaced by allene. Also 0.04 g of palladium acetate were used in association 1.36 g of phenylphosphonic acid and 1.9 g of PPBN.

### EXAMPLE 5 - COMPARATIVE

Example 4 was repeated except that triphenyl phosphine was used as the phosphine ligand instead of PPBN. Also 0.18 g of palladium acetate were used in association 6.32 g of phenylphosphonic acid and 10.5 g of triphenyl phosphine.

At the end of 24 hours palladium metal precipitate was present in the discharged catalyst solution. The results of Examples 4 and 5 are shown in Table 3 as the percentage (w/v) of methyl methacrylate detected in the withdrawn samples.

**TABLE 3**

| LIGAND | REACTION TIME (HOURS) | |
|---|---|---|
| | 2 | 24 |
| PPBN | 0.1 | 1.4 |
| Triphenyl phosphine | 0.0 | 0.5 |

In terms of Relative Rates the results are as shown in Table 3a.

**TABLE 3a**

| LIGAND | REACTION TIME (HOURS) | |
|---|---|---|
| | 2 | 24 |
| PPBN | 7.4 | 6.6 |
| Triphenyl phosphine | 0.0 | 0.5 |

It is thus evident that the conventional phosphine ligand is much less active than PPBN in the presence of allene.

### EXAMPLE 6

Example 1 was repeated except that the individual isomers were used instead of a mixture of the isomers. Also, different amounts of palladium acetate, phenylphosphonic acid and isomers were used as follows: (i) 0.08 g palladium acetate in association with 3.9 g of phenylphosphonic acid and 4.0 g of [3,3,1] isomer; (ii) 0.09 g palladium acetate in association with 3.07 g of phenylphosphonic acid and 4.3 g of [4,2,1] isomer; and (iii) 0.09 g palladium acetate in association with 3.13 g of phenylphosphonic acid and 4.6 g of [4,2,1] isomer. In both experiments using the [4,2,1] isomer the results were approximately the same.

In terms of the Relative Rates, the results of the example are shown in Table 4.

**TABLE 4**

| LIGAND | AFTER 1 HOUR REACTION TIME |
|---|---|
| [3,3,1] | 10.0 |
| [4,2,1] | 45.0 |

### EXAMPLE 7

In this example the long term stability of the [4,2,1] isomer was assessed in the following manner.

In a first run, the procedure of Example 1 was followed using a catalyst having a formulation of 0.03 g palladium acetate in association with 0.89 g of phenylphosphonic acid and 1.3 g of [4,2,1] isomer. After a reaction time of one hour, the entire contents of the autoclave were removed under nitrogen for analysis by gas chromatography. The analysis showed the presence of methyl methacrylate, methanol and unreacted propyne. After removal of volatiles under reduced pressure, a fresh charge of methanol was added and on further analysis the solution was shown to be free from residual methyl methacrylate.

A second run was then performed in a similar manner to the first run but using the catalyst from the first run instead of fresh catalyst. The analysis was repeated at the end of the second run.

Finally, a third run was performed using the catalyst from the second run. Again the analysis was repeated.

The results of the analysis showed that after each run approximately 0.2 % w/v of methyl methacrylate had been formed and that the catalyst had maintained a Relative Rate of 36.

### EXAMPLE 8

In this example the thermal stability of the [4,2,1] isomer was assessed in the following manner.

The procedure of Example 1 was repeated using a catalyst having a formulation of 0.04 g palladium acetate in association with 1.73 g of phenylphosphonic acid and 2.3 g of [4,2,1] isomer, except that the temperature was raised to 90°C. Analysis showed that 1.7% w/v of methyl methacrylate was present at the end of the run and that the catalyst had a Relative Rate of 146.

## Claims

1. A process capable of the low temperature carbonylation of an acetylenically unsaturated compound with carbon monoxide in the presence of an allenically unsaturated compound and a catalytic system which is capable of catalysing the carbonylation of the acetylenically unsaturated compound and which is at least tolerant of the allenically unsaturated compound, the catalytic system being formed from
(a)a palladium compound;
(b)a protonic acid;
(c)an organic phosphine of the form (I) 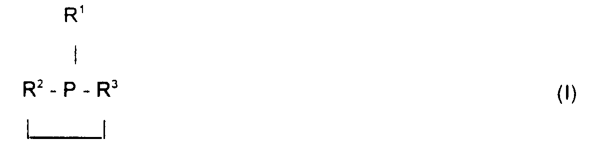
wherein
R¹ is an optionally substituted aryl group;
R² and R³ are joined to form an optionally substituted cyclic moiety comprising at least one optionally substituted alkylene group.

2. A process as claimed in claim 1 wherein the cyclic moiety is a relatively rigid structure which contains from 4 to 15 optionally substituted alkylene groups.

3. A process as claimed in either claim 1 or claim 2 wherein the organic phosphine is one in which the phosphorus atom, R² and R³ form a polycyclic group in which the phosphorus atom is a link on a bridge shared by at least two cyclic groups.

4. A process as claimed in claim 3 wherein the polycyclic groups are selected from 9-phosphabicyclo[4,2,1]-nonane and 9-phosphabicyclo[3,3,1]-nonane.

5. A process as claimed in any one of claims 1 to 4 wherein the group R¹ is selected from anthryl, naphthyl and phenyl and substituted analogues thereof.

6. A process as claimed in claim 5 wherein the group R¹ is phenyl.

7. A process as claimed in any one of claims 1 to 6 wherein the palladium compound is palladium acetate and the protonic acid is phenylphosphonic acid.

8. A process as claimed in any one of claims 1 to 7 wherein the quantity of palladium compound used is such that the molar ratio of palladium to unsaturated compound is from 10⁻⁶:1 to 1:1.

9. A process as claimed in any one of claims 1 to 8 wherein the quantity of organic phosphine used is such that the molar ratio of organic phosphine to palladium compound is from 200:1 to 1:1.

10. A process as claimed in any one of claims 1 to 9 wherein the acetylenically unsaturated compound is one or more unsaturated alkyne having from 2 to 20 carbon atoms.

11. A process as claimed in any one of claims 1 to 10 wherein the allenically unsaturated compound is one or more alkadiene having from 3 to 20 carbon atoms.

12. A process as claimed in any one of claims 1 to 11 wherein the temperature at which the carbonylation is performed is less than 115°C.

## Patentansprüche

1. Verfahren zur Carbonylierung einer acetylenisch ungesättigten Verbindung mit Kohlenmonoxid bei tiefen Temperaturen in Gegenwart einer allenisch ungesättigten Verbindung sowie Katalysatorsystem zur Katalyse der Carbonylierung der acetylenisch ungesättigten Verbindung, das zumindest die allenisch ungesättigte Verbindung toleriert, wobei das Katalysatorsystem aus
(a) einer Palladiumverbindung;
(b) einer protischen Säure;
(c) einem organischen Phosphin der Formel (I) 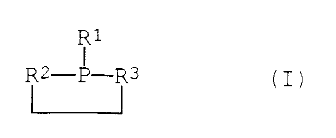
gebildet ist, wobei
R¹ eine gegebenenfalls substituierte Arylgruppe ist;
R² und R³ unter Bildung einer gegebenenfalls substituierten cyclischen Einheit, die mindestens eine gegebenenfalls substituierte Alkylengruppe umfaßt, verknüpft sind.

2. Verfahren nach Anspruch 1, wobei die cyclische Einheit eine relativ starre Struktur hat, die 4 bis 15 gegebenenfalls substituierte Alkylengruppen enthält.

3. Verfahren nach einem der Ansprüche 1 oder 2, wobei beim organischen Phosphin das Phosphoratom, R² und R³ eine polycyclische Gruppe bilden, bei der das Phosphoratom eine Verknüpfung in einer von mindestens zwei cyclischen Gruppen geteilten Brücke ist.

4. Verfahren nach Anspruch 3, wobei die polycyclischen Gruppen aus 9-Phosphabicyclo[4.2.1]nonan und 9-Phosphabicyclo[34.3.1]nonan ausgewählt sind.

5. Verfahren nach einem der Ansprüche 1 bis 4, wobei die Gruppe R¹ aus Anthryl, Naphthyl und Phenyl sowie substituierten Analoga davon ausgewählt ist.

6. Verfahren nach Anspruch 5, wobei die Gruppe R¹ Phenyl ist.

7. Verfahren nach einem der Ansprüche 1 bis 6, wobei die Palladiumverbindung Palladiumacetat und die protische Säure Phenylphosphonsäure ist.

8. Verfahren nach einem der Ansprüche 1 bis 7, wobei die verwendete Menge an Palladiumverbindung derart ist, daß das molare Verhältnis von Palladium zu ungesättigter Verbindung 10⁻⁶:1 bis 1.1 ist.

9. Verfahren nach einem der Ansprüche 1 bis 8, wobei die Menge an verwendetem organischen Phosphin derart ist, daß das molare Verhältnis von organischem Phosphin zur Palladiumverbindung 200:1 bis 1:1 ist.

10. Verfahren nach einem der Ansprüche 1 bis 9, wobei die acetylenisch ungesättigte Verbindung ein ein- oder mehrfach ungesättigtes Alkin mit 2 bis 20 Kohlenstoffatomen ist.

11. Verfahren nach einem der Ansprüche 1 bis 10, wobei die allenisch ungesättigte Verbindung ein Alkadien mit 3 bis 20 Kohlenstoffatomen ist.

12. Verfahren nach einem der Ansprüche 1 bis 11, wobei die Temperatur, bei welcher die Carbonylierung durchgeführt wird, weniger als 115°C beträgt.

## Revendications

1. Procédé capable de réaliser la carbonylation à basse température d'un composé acétyléniquement insaturé avec du monoxyde de carbone en présence d'un composé alléniquement insaturé et d'un système catalytique qui est capable de catalyser la carbonylation du composé acétyléniquement insaturé et qui tolère au moins le composé alléniquement insaturé, le système catalytique étant formé à partir de:
(a) un composé du palladium,
(b) un acide protonique,
(c) une phosphine organique de forme (I): 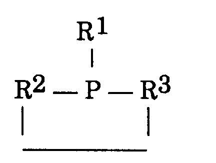
dans laquelle :
R¹ est un groupe aryle éventuellement substitué,
R² et R³ sont réunis pour former une partie cyclique éventuellement substituée comprenant au moins un groupe alkylène éventuellement substitué.

2. Procédé suivant la revendication 1, dans lequel la partie cyclique est une structure relativement rigide qui contient de 4 à 15 groupes alkylène éventuellement substitués.

3. Procédé suivant l'une ou l'autre des revendications 1 ou 2, dans lequel la phosphine organique est une phosphine dans laquelle l'atome de phosphore, R² et R³ forment un groupe polycyclique dans lequel l'atome de phosphore est une liaison sur un pont partagé par au moins deux groupes cycliques.

4. Procédé suivant la revendication 3, dans lequel les groupes polycycliques sont choisis parmi le 9-phosphabicyclo-[4.2.1]-nonane et le 9-phosphabicyclo-[3.3.1]-nonane.

5. Procédé suivant l'une quelconque des revendications 1 à 4, dans lequel le groupe R¹ est choisi parmi les groupes anthryle, naphtyle et phényle et leurs analogues éventuellement substitués.

6. Procédé suivant la revendication 5, dans lequel le groupe R¹ est le groupe phényle.

7. Procédé suivant l'une quelconque des revendications 1 à 6, dans lequel le composé du palladium est l'acétate de palladium et l'acide protonique est l'acide phénylphosphonique.

8. Procédé suivant l'une quelconque des revendications 1 à 7, dans lequel la quantité de composé du palladium utilisé est telle que le rapport molaire du palladium au composé insaturé est compris entre de 10⁻⁶ :1 et 1:1.

9. Procédé suivant l'une quelconque des revendications 1 à 8, dans lequel la quantité de phosphine organique utilisée est telle que le rapport molaire de la phosphine organique au composé du palladium est compris entre 200:1 et 1:1.

10. Procédé suivant l'une quelconque des revendications 1 à 9, dans lequel le composé acétyléniquement insaturé comprend un ou plusieurs alcynes insaturés ayant de 2 à 20 atomes de carbone.

11. Procédé suivant l'une quelconque des revendications 1 à 10, dans lequel le composé alléniquement insaturé comprend un ou plusieurs alkadiènes ayant de 3 à 20 atomes de carbone.

12. Procédé suivant l'une quelconque des revendications 1 à 11, dans lequel la température à laquelle est réalisée la carbonylation est inférieure à 115°C.
